Europäisches Patentamt

European Patent Office

Office européen des brevets

(11) Publication number: **0 399 777 A2**

## EUROPEAN PATENT APPLICATION

(21) Application number: 90305529.1

(22) Date of filing: 22.05.90

(51) Int. Cl.5: **C12N 15/27, C12P 21/02, C07K 7/08, A61K 37/02**

(30) Priority: 23.05.89 US 356006

(43) Date of publication of application:
28.11.90 Bulletin 90/48

(84) Designated Contracting States:
AT BE CH DE DK ES FR GB GR IT LI LU NL SE

(71) Applicant: **Choudhury, Chandra**
**1610 Robert E. Lee Blvd., Apt. 29**
**New Orleans, Louisiana 70122(US)**

(72) Inventor: **Choudhury, Chandra**
**1610 Robert E. Lee Blvd., Apt. 29**
**New Orleans, Louisiana 70122(US)**

(74) Representative: **Brown, John David et al**
**FORRESTER & BOEHMERT**
**Widenmayerstrasse 4/I**
**D-8000 München 22(DE)**

(54) Hematopoietic growth factor derived from T lymphocytes and methods of use therefor.

(57) A T cell-derived colony stimulating factor (TC-CSF) may be isolated from media conditioned with T lymphocyte cells. TC-CSF stimulates formation of colonies composed of granulocytes, macrophages, megakaryocytes and mixed colonies of granulocytes and macrophages. Anion exchange chromatography may be employed in conjunction with gel filtration to isolate murine TC-CSF having a molecular mass of about 55 Kd.

EP 0 399 777 A2

## HEMATOPOIETIC GROWTH FACTOR DERIVED FROM T LYMPHOCYTES AND METHODS OF USE THERE-FOR

### Background

The present invention relates in general to colony stimulating factors and specifically relates to colony stimulating factors derived from T cells.

Mediators of proliferation and differentiation for hematopoietic progenitor cells of all lineages are frequently present in the same conditioned medium. Although these secreted growth factors have similar molecular mass (usually between 25 Kd to 35 Kd) and may have apparently overlapping functional properties, they are products of distinct genes.

Hematopoietic growth factors include factors which stimulate development of certain hematopoietic cell lineages: IL-3, which has multi-lineage activity; GM-CSF, which predominantly stimulates granulocyte and macrophage colony formation, although, under certain conditions, it may stimulate megakaryocytic lineage cells as well; CSF-1, which stimulates only macrophage colonies; and G-CSF, which stimulates only granulocyte colony formation. None of these growth factors have a molecular mass exceeding about 30 Kd.

Hematopoietic growth factors may be useful in treating immune-compromised patients, including AIDS patients and certain types of cancer patients. Accordingly, it is desirable to obtain additional hematopoietic growth factors for existing and new applications.

### Summary of the Invention

The present invention provides a purified and isolated nucleic acid encoding a TC-CSF and described by a nucleotide sequence selected from the group consisting of a nucleotide sequence encoding a TC-CSF, a nucleotide sequence which encodes the sequence of amino acids Met-Pro-Asn-Leu-Gly-Ile-Phe-Ala-Asp-Thr-Met-Pro-Glu-Asp-Ala-Gly-Asn-Ser-Val, a nucleotide sequence which hybridizes with any 20 sequential nucleotides encoding the sequence of amino acids Met-Pro-Asn-Leu-Gly-Ile-Phe-Ala-Asp-Thr-Met-Pro-Glu-Asp-Ala-Gly-Asn-Ser-Val or with the complement thereof, a nucleotide sequence which hybridizes under strict conditions with any 20 sequential nucleotides in a TC-CSF or with the complement thereof, and a nucleotide sequence which encodes an epitope encoded by 6 sequential amino acids in a TC-CSF or in the amino acid sequence Met-Pro-Asn-Leu-Gly-Ile-Phe-Ala-Asp-Thr-Met-Pro-Glu-Asp-Ala-Gly-Asn-Ser-Val.

The present invention further provides a vector including a nucleic acid according to the present invention, and a cell, preferably a eukaryotic cell, including such a vector or including a nucleic acid according to the present invention at a location or in a multiplicity in which it does not occur in nature.

The present invention also provides an isolated polypeptide having a biological or immunological property of a T cell-derived colony stimulating factor and having an amino acid sequence in which at least 6 sequential amino acids are identical to 6 sequential amino acids in a TC-CSF, particularly wherein the polypeptide includes an epitope of TC-CSF.

An isolated TC-CSF polypeptide according to the present invention may be selected from the group consisting of human TC-CSF, mouse TC-CSF, rat TC-CSF, bovine TC-CSF, canine TC-CSF, feline TC-CSF, ovine TC-CSF, ape TC-CSF, avian TC-CSF, and porcine TC-CSF, especially a TC-CSF having a molecular mass of about 55 Kd, and may include an amino acid sequence comprising Met-Pro-Asn-Leu-Gly-Ile-Phe-Ala-Asp-Thr-Met-Pro-Glu-Asp-Ala-Gly-Asn-Ser-Val. The TC-CSF may be an expression product of a cell including a vector having DNA encoding TC-CSF or of a cell including a nucleic acid according to the present invention at a location or in a multiplicity in which it does not occur in nature. The isolated TC-CSF polypeptide may further include a diluent, adjuvant or carrier, preferably wherein the diluent comprises an isotonic buffer or pharmaceutical grade water for injection to form a pharmaceutical composition.

Any TC-CSF or nucleic acid according to the present invention not directly provided herein may be obtained according to procedures well known to those skilled in the art, including obtaining the complete amino acid sequence of the TC-CSF provided herein and screening DNA libraries with polynucleotide probes based thereon, and including identifying cells expressing TC-CSF by using a labeled antibody or oligonucleotide according to the present invention, isolating mRNA therefrom and preparing CDNA from the TC-CSF mRNA.

The isolated polypeptide may also include a label or reporter group or may be bound to a support. The isolated TC-CSF polypeptide according to the present invention may be associated with a support and an anti-TC-CSF antibody in a diagnostic kit.

In addition, the present invention provides an isolated ligand specifically binding a TC-CSF, which ligand may be a TC-CSF receptor. Such a TC-CSF receptor may be identified using labeled TC-CSF according to the pressent invention and may be isolated using purified TC-CSF according to the present invention by techniques well known to those skilled in the art. Alternatively, the ligand may be serum or an isolated monoclonal or polyclonal antibody exhibiting a specific immunoreactivity with a TC-CSF, in particular exhibiting a specific immunoreactivity with an expression product of a cell containing a vector having DNA encoding a TC-CSF or a cell including a nucleic acid according to the present invention at a location or in a multiplicity in which it does not occur in nature.

A purified and isolated antigen is provided according to the present invention exhibiting immunological characteristics such that it specifically reacts with a monoclonal or polyclonal antibody against a TC-CSF, and the present invention also includes an immortalized cell line, such as a hybridoma, producing a monoclonal antibody to TC-CSF.

A process for purifying TC-CSF according to the present invention includes the steps of applying cell culture supernatant containing TC-CSF to an anion exchange column, and collecting a fraction containing a TC-CSF from the HPLC anion exchange column, especially wherein the applying step includes the step of introducing the TC-CSF onto a column having a quaternary ammonium group bound to a support. The purification process may further include the steps of introducing the TC-CSF into a gel filtration column and retaining fractions comprising TC-CSF.

A method for production of a T cell-derived colony stimulating factor (TC-CSF) according to the present invention includes the step of culturing a medium enriched in cells containing a gene encoding TC-CSF under conditions which permit expression of the gene and isolating TC-CSF from the medium or cells.

A method of treatment according to the present invention includes administering to a patient in need of therapy an effective amount of a pharmaceutical composition according to the present invention, and preferably includes administering to a patient in need of therapy an effective amount of a pharmaceutical composition including a TC-CSF.

## Detailed Description

"Operably linked" as used herein refers to a structural connection which permits the normal function (as understood by those skilled in the art) of the components to be performed. Thus, a coding sequence is "operably linked" to a control sequence when the coding sequence is located on the same or a complementary DNA strand and its transcription is affected by the control of this sequence.

"Exogenous DNA" as used herein refers to DNA in a host cell in which it is not found in nature or under the control of a promoter to which it is not operably linked in nature or in a number of copies per cell which does not occur in nature.

As used herein, a medium is "enriched" in a kind of cell if it contains only that kind of cell or it it contains a higher proportion of the kind of cell relative to other cells in the medium.

As used herein, the term "cell" includes progeny. Thus, "transformed cells" include a cell transformed with a vector and cells derived therefrom without regard for the number of cell division even though progeny may not be genetically identical due to deliberate or inadvertent mutations.

"Expression system" herein includes DNA encoding a polypeptide to be expressed operably linked to DNA controlling transcription of the DNA sequence, so that a host transformed with such DNA expresses the encoded polypeptides. The expression system may be included in a vector called an "expression vector" or may be integrated into the host chromosome.

"Post-translational modification" as used herein includes glycosylation but may also involve proteolysis, phosphorylation, acylation, sulfation, $\gamma$-carboxylation or $\beta$-hydroxylation.

Included within the scope of the present invention is TC-CSF expressed in a cell including: TC-CSF having the glycosylation and amino acid sequence of naturally-occurring TC-CSF; human TC-CSF and TC-CSF polypeptides of other animal species, such as bovine TC-CSF, equine TC-CSF, porcine TC-CSF, ovine TC-CSF, canine TC-CSF, murine TC-CSF, feline TC-CSF, without limitation thereto; deglycosylated or nonglycosylated forms of TC-CSF polypeptides; and analogs of TC-CSF, particularly immunologically and biologically active analogs of TC-CSF.

"Analogs of TC-CSF" include, for example, deletions from, or insertions or substitutions of, residues within the amino acid sequence of TC-CSF retaining at least consecutive 18 nucleotides in the nucleotides in the nucleotide sequence or 6 consecutive amino acids in the amino acid sequence of TC-CSF and retaining a biological or immuno logical activity of TC-CSF and an overall amino acid and nucleotide sequence homology of at least 80% with TC-CSF. Combinations of deletion, insertion and substitution may also be made to arrive at the final construct, provided that the final construct possesses an activity of TC-CSF. An "activity" of TC-CSF may be an immunological cross-reactivity with naturally-occurring TC-CSF or may be a biological activity (e.g., a colony stimulating activity) of TC-CSF. It is preferred that mutations made in DNA encoding TC-CSF do not place the sequence out of reading frame and it is further preferred that they do not create complementary regions that could produce secondary mRNA structure. Analogs also include polypeptides exhibiting a post-translational modification which they do not occur in a nature.

Analogs include amino-terminal and carboxyl-terminal fusion peptides of from one residue to polypeptides of any length wherein the analog possesses an activity of TC-CSF, as well insertions of one more amino acid residues within the polypeptide. Such insertions may be of any length which does not prevent the analog from possessing an activity of TC-CSF but as preferably and amino acid in length.

Natural sequence TC-CSF or analogs and variants of TC-CSF may be prepared by direct chemical synthesis of polypeptide or by expression of DNA prepared by site-directed mutagenesis of TC-CSF DNA or by chemical synthesis of oligonucleotide and assembly of the oligonucleotide by any of a number of techniques prior to expression in a host cell. [See, e.g., Caruthers, U.S. Patent No. 4,500,707: Balland et al., Biochimie, 67 725-736 (1985); Edge et al., Nature, 292, 756-762 (1981)]. Messenger RNA encoding TC-CSF or an analog thereof may also be expressed in vitro. Changes in activation levels are measured by the appropriate assay. Modifications of such protein properties as redox or thermal stability, hydrophobicity, susceptibility to proteolytic degradation, or the tendency to aggregate with carriers or into multimers are assayed by methods well known to those of ordinary skill in the art.

Prokaryotic microorganisms (such as bacteria) and eukaryotic microorganisms (such as yeast) may be employed as host cells according to the present invention. S. cerevisiae, or common baker's yeast, is the most commonly used among eukaryotic microorganisms, although a number of other strains are commonly available. For expression in bacteria and yeast, cloning and expression vectors are well known to those skilled in the art, such as lambda phage and pBR322 in E. coli and YRp7 in S. cerevisiae.

Cells derived from multicellular eukaryotes may also be used as hosts. Cells from vertebrate or invertebrate eukaryotes may be used, and those skilled in the art know of appropriate expression vectors for use therein, such as SV40 vectors for mammalian host cells, NPV vectors for invertebrate host cells and Ti vectors for plant cells.

The compositions according to the present invention are formulated in accordance with standard procedures to be adapted for parenteral administration to humans according to techniques known by those skilled in the art.

Typically, the compositions for intravenous administration are solutions of the sterile derivative in sterile isotonic aqueous buffer. Where necessary, the composition also includes a solubilizing agent, or stabilizing or carrier agent for the complex. In general, the complex is supplied in unit dosage form, for example, as a dry powder or water-free concentrate in a sealed container such as an ampoule. For administration by infusion, the complex may be dispensed from an infusion bottle containing sterile pharmaceutical grade water for injection. For administration by injection, the complex may be dispensed from a vial of sterile water for injection. The injectable or infusible composition may be made up by mixing the ingredients prior to administration.

The effective amount TC-CSF administered will depend on many factors, including the amount of TC-CSF required and the speed with which it is required.

More specific descriptions of methods, materials, and products according to the present invention appear in the following Examples.

## Example 1

## Process for Isolation of TC-CSF

TC-CSF may be prepared by stimulation of a murine T lymphocyte cell line, the EL-4 cell line (available as Culture No. ATCC TIB 181 from the American Type Culture Collection, Rockville, Maryland) with phorbol 12-myristate 13-acetate (PMA) (Sigma Chemical Company, St. Louis, Missouri). The cells may be grown in RPMI-1640 (GIBCO, Grand Island, New York) containing 5% bovine serum albumin or 5% fetal calf serum at 37° C. in an atmosphere of 5% $CO_2$ in air. For stimulation with PMA, the EL-4 cells may be centrifuged at 500 g for 10 minutes and then resuspended in RPMI-1640, 1% fetal calf serum (FCS) (GIBCO), and 56 units/ml penicillin (GIBCO) 56 $\mu$g/ml streptomycin and 10 $\mu$g/ml glutamine. The cell concentration is then adjusted to 1 X 10$^6$ cells per ml. The volume of the cell suspension may be measured and to this volume a calculated amount of PMA may be added to give a final concentration of 10 ng/ml PMA. The stimulation is preferably done in sterile glass containers with approximately 150 ml of cell suspension in each 500 ml capacity glass container. The cell suspension may be incubated at 37° C. for 2 days in an atmosphere of 5% $CO_2$ in air. Following the incubation the cells may be removed by centrifugation at 2000 rpm for 10 minutes and the supernatant containing the growth factor may be stored at 4° C.

Any residual PMA in the supernatant may be removed by treatment with activated dextran by the following method. For each 100 ml of supernatant 0.3 g Norit A alkaline (Fisher, Pittsburg, Pennsylvania) and 1.2 g Dextran T 10 (Pharmacia, Piscataway, New Jersey) may be suspended in 200 ml phosphate buffered saline (PBS) plus 1% fetal calf serum or in 200 ml RPMI-1640 and 1% FCS. The Norit A and Dextran may be mixed in half the PBS, then poured into 200 ml capacity centrifuge bottles and centrifuged at 2000 rpm for 5 minutes. The supernatant is discarded and the remaining PBS is added to the Norit A and Dextran, the procedure is repeated and the supernatant discarded. Then following these two washes with Norit A and Dextran the growth factor containing culture supernatant may be added to the Norit A plus Dextran, mixed and centrifuged at 2000 rpm for 5 minutes. The growth factor conditioned medium supernatant is poured off and then may be filtered through a 0.45 $\mu$ filter as available from Millipore Corporation, Bedford, Massachusetts.


## Example 2


## Bioassay for TC-CSF


The hematopoietic growth stimulating activity of a conditioned medium may be tested utilizing the agar clonal assay. Following stimulation with a growth factor, hematopoietic progenitor cells form colonies in the agar cultures. Hematopoietic cells utilized for the bioassay may be derived from bone marrow or spleen of rodents such as C57B1/6J mice.

Following euthanasia of a mouse the femur is dissected out and bone marrow cells may be flushed out with RPMI-1640 using a syringe and needle. The cells may be dispersed by pipetting, and a single cell suspension may be derived. The viable cell count maybe obtained by Trypan blue exclusion of cells counted in a hemocytometer. Bone marrow cells may be plated at 1 X 10$^5$ or 5 X 10$^4$ cells per plate. Duplicate or triplicate plates may be set up. The cell cultures may be set up in 0.3% agar (Bactoagar, GIBCO) in Dulbecco's medium (GIBCO) with 20% horse serum (GIBCO), supplemented with 56 units/ml penicillin and 56 $\mu$g/ml streptomycin. TC-CSF-conditioned media maybe added at 10% V/V to the agar cultures. The cultures are then incubated at 37° C. for seven days in an atmosphere of 10% $CO_2$ in air.


## Example 3


## Bioactivity of TC-CSF


The colonies formed following stimulation with conditioned media containing TC-CSF may be enumerated microscopically at 40 X magnification. Colony forming unit-culture (CFU-C) colonies contained 50 or more cells.

The lineage specificity of CFU-C colonies may be determined by morphological assessment of the cells comprising each colony. Intact agar cultures may be dehydrated on glass slides and stained for acetylcholinesterase. A counterstain that may be utilized for nuclei is methyl green. The colonies may then be examined microscopically, the nuclear configuration, cell shape and size allow differentiation between the cell lineages such as granulocytic, mononuclear, fibrocytic stromal and megakaryocytic. In addition the megakaryocyte cytoplasm stains brown due to the presence of acetyl cholinesterase. Clusters of three or more megakaryocytes may be taken as representing a megakaryocyte colony.

TC-CSF-containing conditioned media, as prepared in procedure of Example 1 stimulate the formation of approximately $90 \pm 20$ CFU-C colonies (mean $\pm$ 2 SD) and $20 \pm 4$ CFU-M (megakaryocyte colonies), per $1 \times 10^5$ bone marrow cells. Morphological analysis of the colonies reveals that TC-CSF-generated colonies are composed of: granulocytes; macrophages; megakaryocytes; mixtures of granulocytes and macrophages; mixed colonies which were very large compared with the other colonies, (mixed colonies consist of more than 150 cells entities composed of granulocytes, macrophages and megakaryocytes); and fibrocytic stromal cell colonies.

## Example 4

## Isolation of TC-CSF

Protein fractions contained in the conditioned media derived as in the final product of Example 1 may be separated by high pressure liquid chromatography (HPLC) on an ion-exchange column DEAE-5PW, 7.5 mm X 7.5 cm, available from Waters Associates, Milford, Massachusetts. A phosphate buffered saline gradient may be utilized for elution with a gradient range from 10 mM NaCl to 1 M NaCl. The fractions may be collected at a flow rate of 0.5 ml/min and then each fraction may be assayed for colony stimulating activity as described in Examples 2 and 3. Bioassay for colony stimulating activity of the fractions demonstrated peak activity with a 0.23 M NaCl gradient. There may be more than a 90% recovery of activity compared to the initial conditioned medium. The activity of the TC-CSF at 0.23 M NaCl gradient serves to distinguish the TC-CSF molecule from Interleukin-3 (IL-3), which may also stimulate hematopoietic colony formation, because IL-3 does not bind to DEAE and runs through a DEAE column before the NaCl gradient fractions.

The TC-CSF molecule may be separated from other molecules based upon the size of the TC-CSF molecule obtained by gel filtration. A molecular sieve column protein Pak 125 (size cut-off for native globular protein, 2,000 to 80,000 daltons), 7.8 mm X 30 cm, from Waters Associates may be utilized with HPLC at a flow rate of 0.5 ml/min. A variable wavelength spectrophotometer and an integrator/recorder may be attached to the detector set at 280 nm to record and integrate the chromatograms. The fractions may be collected in PBS (pH 7.3), and then each fraction assayed for colony stimulating factors. Assays of the fractions indicated activity was present in three fractions, and corresponding peaks of optical density at 280 nm indicated the presence of protein in the active fractions. A first fraction corresponds to a molecular mass (MW) of 65 to 70 kilodaltons (Kd), consistent with the trace amount of bovine serum albumin and bovine fetal albumin present in the conditioned media. A second fraction may have peak activity and corresponds to MW 50 to 55 Kd. A third fraction with minimal activity corresponds to MW 25 to 30 Kd.

## Example 5

## Molecular Weight Assessed By Electrophoretic Mobility

The bioactive second fraction derived after HPLC gel filtration in Example 4 and the unseparated conditioned media may be further analyzed by polyacrylamide gel electrophoresis (PAGE). Either polyacrylamide urea gel electrophoresis at pH 9.9 in a reducing buffer of 50 mM 2-mercaptoethanol (2-ME) and 10 M urea, or SDS-PAGE in 0.3% SDS, 7.5% acrylamide and 10 mM 2-ME in glycerol buffer my be employed. Samples may be run 50 $\mu$l per lane with growth factor media or molecular weight markers purchased from Pharmacia. Lanes run with molecular weight markers and designated samples respectively, may be stained by silver stain to identify the protein molecule according to electrophoretic mobility. The TC-CSF demonstrates an electrophoretic mobility in PAGE under reducing conditions corresponding to a MW of about 55 Kd.

Confirmation of bioactivity in the molecule identified by electrophoretic mobility may be obtained by running lanes containing the sample under investigation. Following electrophoresis the entire lane may be sectioned into 1 mm wide slices starting from the cathodal end and proceeding towards the anodal end. The protein from each 1 mm slice may be eluted in 1 ml PBS at pH 7.2 and then assayed for colony stimulating factor. Adjacent lanes with appropriate molecular weight markers may be stained to determine the electrophoretic mobility of the bioactive molecule, which corresponded to a MW or about 55 Kd.

Following bioassay of the protein derived from the gel slice, a portion of the eluate may be re-run on SDS-page and following stain with silver stain demonstrate a single protein band at about 55 Kd. Thus confirmation may be derived that bioactive TC-CSF molecule has a MW about 55 Kd, demonstrated by electrophoretic mobility under reducing conditions.

## Example 6

## Protein Content and Potency of TC-CSF

The protein content of the TC-CSF containing media and that of the fractions may be measured by microassay procedure. Spectrophotometric analysis is possible at 595 nm following reaction with protein assay dye reagent available from Bio-Rad, Richmond, California, and the protein content may be determined from a standard curve prepared with BSA.

After the least measurable protein content has been estimated, further dilutions may be made and the protein content calculated. Assays of dilutions of the fractions correlated with 10 to 14 nanograms of protein stimulate 5 to 8 CFU-C per $1 \times 10^5$ bone marrow cells. This corresponded to activity at a 1 to 2 picomolar concentration of TC-CSF, an indication of the potency of the growth factor.

## Example 7

## Production of an Antibody Against TC-CSF and Assays

Animals such as Balb/c mice or Lewis rats may be immunized by intraperitoneal injection of a TC-CSF-containing fraction obtained following HPLC column separation as in Example 4. The growth factor-containing fraction may be combined with Freund's adjuvant (one part in three) and injected into the animals, followed by a booster dose 4 weeks later, after which the animals may be sacrificed and the sera obtained.

In an ELISA assay, the second HPLC-derived fraction from Example 4 containing the TC-CSF may be used as antigen, and diluted in coating buffer to give a final protein ooncentration of about 0.5 $\mu$g/ml (coating buffer is prepared by dissolving 0.795 g anhydrous $Na_2CO_3$ and 1.466 g $NaHCO_3$ in 450 ml of deionised water at pH 9.6). Next, 100 $\mu$l of the diluted antigen may be added to each well of micro ELISA plate (Corning) and then the plates may be placed in a covered container at 4° C. overnight. The plates may then be removed and each well washed 3 times with 200 $\mu$l of PBS/Tween-20TM (stock solution prepared by adding 1.5 ml Tween-20TM to 1000 ml PBS). The antibody containing sera is added 1:50 in PBS/Tween-20TM and 100 $\mu$l of the diluted antiserum may be added to the wells and incubated for one hour at 37° C. The wells may then be washed three times and 100 $\mu$l of anti-mouse IgG alkaline phosphatase conjugate (Sigma) which had been diluted 1:1000 in stock PBS/Tween-20TM may be added to each well. Following a further one hour incubation at 37° C., the wells may be washed three times and 100 $\mu$l volume of diethanolamine substrate (Sigma) may be placed in each well. A positive reaction is observed by yellow color formation within a half hour, indicating specific reaction with TC-CSF (antigen) and antibody contained in the sera. Following addition of the antibody sera in dilutions of 1:1000, about 56% of the colony stimulating activity of the TC-CSF fraction is neutralized.

Sera from immunized, antibody-producing animals tests positive when screened against TC-CSF-containing fraction by the ELISA technique using reagents obtained from Sigma Chemical Company. Control sera from uninjected mice and those injected with Freund's adjuvant alone tested negative. The antibody may be tested for neutralizing activity in the bioassay with the TC-CSF containing growth factor providing colony stimulating activity.

In a Western blot analysis, the HPLC gel filtered TC-CSF of the second fraction of Example 4 may be run on SDS-PAGE eletrophoresis and then may be placed in the transblot system (Hoeffer Scientific, San Francisco, California). The protein bands may then be transferred onto nitrocellulose sheet which may then be incubated for one hour in blocking buffer (Sigma). Antibody may then be added and a further one hour incubation done. Following three washes anti-mouse IgG alkaline phosphatase conjugate (Sigma) may be added, and, following an hour incubation and five washes in PBS/Tween-20™, the substrate Naphthol As-Mx phosphate sodium salt (Sigma) may be added. A positive reaction is determined by Immunostain with Fast red substrate (Sigma). The protein bands in adjacent lanes may be visualized by staining for protein such as with Amido Black.

Thus, the specificity of the antibody against the TC-CSF is also observed in a Western blot analysis. The antibody strongly react with the protein band at 55 Kd. Reaction was also seen with bands at 67 Kd and 65 Kd which corresponded to fetal calf albumin and bovine serum albumin respectively, the albumin being a carrier protein for the growth factor molecules. No reaction is seen with proteins below 55 Kd MW.

The antibody sera may be tested against IL-3 to look for cross-reactivity. No neutralization of IL-3 activity is observed. This serves to distinguish TC-CSF from IL-3.

Antibody against a TC-CSF msy be purified from sera using techniques well known to those skilled in the art and including passing TC-CSF antibody-containing fluid over a column to which is bound TC-CSF, such as material derived from the active second fraction of Example 4. Similarly, TC-CSF may be purified by a procedure including a step of passing a fluid containing TC-CSF over a column to which anti-TC-CSF antibody is bound.

Example 8

Effect of Other Antibodies On TC-CSF

Specific antibodies against several growth factors implicated in hematopoiesis may be tested against the TC-CSF growth factor to assess for any neutralization, using the agar clonal assay as described in Example 2 and 3. Some of these antibodies are directed against IL-3, GM-CSF, BSF-1 and the IL-2 receptor. Monoclonal antibodies may be tested in a 1:1 dilution with the TC-CSF growth factor fraction and animal sera containing antibody in a dilution of 1:500. The specificity of the antibodies and thus ability to neutralize the respective growth factors or receptor is established previously. [See, for example anti-IL-2 receptors antibodies 3C7 and 7D4, Ortega et al., J. Immunol., 133, 1970 (1984); Malek et al., J. Immunol., 133, 1976 (1984); anti-murine recombinant GM-CSF, Mochizuki Dy et al., J. Immunol., 136, 3706 (1986); for anti IL-3, monoclonal antibody 19B3.1, Abrams et al., J. Immunol., 140, 131 (1988); anti-BSF-1, monoclonal antibody 11B11, Ohara et al., Nature, 315, 333 (1985).] No neutralizing activity by these antibodies on the TC-CSF may be observed. This result illustrates that the TC-CSF is distinct from these other growth factors.

Example 9

Amino Acid Composition TC-CSF

The amino acid composition of a TC-CSF sample may be prepared in the following manner. Following gel electrophoresis as in Example 5, a TC-CSF-containing gel may be blotted on to a PVDF blot membrane at 90 volts for 24 minutes. The TC-CSF protein band may be identified by staining with Coomasie blue and the band excised and used in a Waters Pico Tag amino acid analysis system. The method involves hydrolysis. and precolumn derivatization of the sample followed by reverse phase HPLC. It is based on the application of the phenylisothiocyanate (PITC) chemistry developed by Edman for amino terminal residue sequencing of proteins using PITC as a tagging reagent in the precolumn derivatization of protein and peptide hydrolysates. The following protocol may be employed: 1) collect and dry the samples; 2) hydrolysis at 110°C. for 24 hours; 3) dry the sample further; 4) derivatization; 5) liquid chromatography and 6) data analysis.

An example of the data derived is given in Table 1. It should be noted that, in this determination, the percent of sample injection in the final volume is 50%. The amino acids tryptophan and cysteine are destroyed during acid hydrolysis.

Table 1

| Amino Acid | Picomoles | Percent | Predicted Number in TC-CSF |
|---|---|---|---|
| Asx | 81.60 | 7.36 | 41 |
| Glx | 138.52 | 12.49 | 69 |
| Ser | 77.18 | 6.96 | 38 |
| Gly | 121.74 | 10.98 | 60 |
| His | 23.35 | 2.11 | 11 |
| Arg | 40.14 | 3.62 | 20 |
| Thr | 39.14 | 3.53 | 20 |
| Ala | 65.98 | 5.95 | 33 |
| Pro | 42.44 | 3.83 | 21 |
| Tyr | 36.49 | 3.29 | 18 |
| Val | 43.77 | 3.95 | 22 |
| Met | 10.59 | 0.96 | 5 |
| Cystine | 36.31 | 3.27 | 18 |
| Ile | 70.09 | 6.32 | 35 |
| Leu | 32.90 | 2.97 | 16 |
| Phe | 125.43 | 11.31 | 62 |
| Lys | 123.34 | 11.12 | 61 |

The amino acid composition data supports the results reported in other Examples indicating the TC-CSF has a M.W of about 55 Kd, and is distinct from other known hematopoietic growth factors.

Example 10

Amino Acid Sequencing

An Applied Biosystems sequencer (Applied Biosystems, Foster City, California) automates the determination of amino acid sequences of proteins. A sample may be prepared in the following manner. Following gel electrophoresis as in Example 5, the gel may be blotted on to PVDF blot membrane at 90 volts for 24 minutes. The TC-CSF protein band may be identified by staining with Coomasie blue and the band excised and placed in a column of the sequencer for sequencing following the procedure recommended by the manufacturer. The sequencer controller may then collect and analyze the chromatographic data to interpret the information.

Interpretation of chromatographic data on amino acid sequencing of TC-CSF suggests an amino terminal residue sequence of:

Met-Pro-Asn-Leu-Gly-Ile-Phe-Ala-Asx-Thr-Met-Pro-Glu-Asp-Ala-Gly-Asn-Ser-Val

Example 11

9

## Differentiation of TC-CSF From Other Known Hematopoietic Growth Factors

The growth factor TC-CSF according to the present invention has a MW of 55 Kd and multi-lineage activity. As noted above, none of IL-3, GM-CSF, CSF-1, and G-CSF have a molecular mass exceeding about 30 Kd. The activity of this growth factor, is not inhibited by specific antibodies against IL-3, GM-CSF, BSF-1 and anti-IL-2 receptor. Comparison of protein conformation and amino acid distribution has been made with albumin, CSF-1, GM-CSF and IL-3 with no demonstration of homology.

Furthermore a partial sequence of the TC-CSF has been compared with albumin, CSF-1, GM-CSF, G-CSF, IL-3, BSF-1, BSF-2, IL-6 and neuroleukin, with no demonstration of proper homology. Neuroleukin was included because it is a T cell lymphokine which has a MW of 56 Kd, although no hematopoietic growth factor activity has been demonstrated by this molecule.

The partial sequence of TC-CSF was compared against a data base consisting of over 1 million residues in 4749 sequences. Highest scores were obtained with the coat protein of bacteriophage q-be, fibronectin and viral polyprotein. This suggests that TC-CSF may have some similarities with these proteins but is not identical to them. It also serves to distinguish TC-CSF further from other known growth factors which may be implicated in hematopoiesis.

## Example 12

## Construction of TC-CSF Oligonucleotides

Oligonucleotides encoding construction of TC-CSF oligonucleotides may be synthesized using the above amino acid sequence by the procedure of Caruthers. U.S. Patent No. 4,415,732 and deoxyribonucleic acid and ribonucleic acid probes and primers for site-directed mutagenesis, or (e.g., for use in polymerase chain reaction assay or amplification procedure) may be made by in vitro transcription from them.

Plasmids including DNA sequences according to the present invention may be labeled with a radioactive isotope or with a non-radioactive chemical tag and used as probes. Such plasmids may also be used to synthesize the labeled RNA probes. The labeled probes may be used to detect the presence of homologous DNA sequences and/or mRNA sequences encoded by these DNA sequences in cells of any species, for example by the Southern or Northern hybridization procedure or by dot blot or slot blot hybridization or by in situ hybridization techniques. Stringent hybridization conditions for hybridization assays according to the present invention may generally be defined as reactions functionally equivalent to hybridization carried out in 4 X SSC and 0.5% SDS at a temperature of 65° C. in the last wash.

Nonstringent hybridization conditions, for identifying TC-CSF encoding DNA by screening cell libraries may be determined by principles known to those skilled in the art of cloning homologous genes.

Oligonucleotides according to the present invention may be bound to beads or may be labeled for use in assays by methods well understood by those skilled in the art.

## Example 13

## TC-CSF Peptides and Antibodies Thereto

Peptides corresponding to different portions of TC-CSF proteins, preferably 12-20 amino acid residues in length, may be chemically synthesized by solid-phase methods. Any TC-CSF polypeptides or analogs according to the present invention may be used to elicit specific polyclonal and monoclonal antibodies [Lerner, Nature, 299, 592-596 (1982); Niman et al., Proc. Natl. Acad. Sci. (USA), 80, 4949-4953 (1983)]. The amino acid sequence of TC-CSF facilitates the design of immunogenic peptides corresponding to suitable immunogenetic regions which may be determined according to procedures well known to those skilled in the art [See, e.g., Novotny et al., Proc. Natl. Acad. Sci. (USA), 83, 226-230 (1986) and Van Regenmortel, Trends Biochem. Sci., 11, 36-39 (1986)].

Example 14

Recombinant Expression of TC-CSF

Complete and partial TC-CSF gene products and analogs may be expressed in bacteria, yeast or mammalian expression systems obtained using products based on amino acid sequences derived from isolated TC-CSF according to the present invention by inserting the DNA encoding TC-CSF into an expression vector, for example a plasmid, phage or viral expression vector [Vieira et al., Gene, 19, 259-268 (1982); Young et al., Proc. Natl. Acad. Sci. (USA), 80, 1194-1198 (1983); Bitter et al., Gene, 32, 263-274 (1984); Cepko et al., Cell, 37, 1053-1062 (1984); and Gorman et al., Mol. Cell. Biol., 2, 1044-1051 (1982)].

The expressed proteins or polypeptides may be purified, for example in Example 4, and used to raise specific antibodies (an epitope comprising about 6 or more amino acids as encoded by 18 or more nucleotides, respectively) or as ligands for antibodies such as may be employed in an immunoassay.

The above data and Examples establish that the TC-CSF is a novel hematopoietic growth factor. Potential uses for TC-CSF include: 1) delineation of the regulation of hematopoiesis; 2) identification of disease states in which de-regulation occurs; 3) stimulation of hematopoiesis (this may be in vitro or in vivo, particularly patients who may have suppression of hematopoiesis either due to disease or secondary to chemotherapeutic agents being used for treatment of a maligancy); 4) gene therapy where introduction of this gene into cells may have beneficial effects; 5) other uses may be considered, such as treatment of immunocompromised patients who concomitantly have low blood counts; and stimulation of hematopoietic cells into a cell cycle phase such that cell cycle phase specific chemotherapeutic agents may then act more effectively.

Although the present invention is described herein in terms of a preferred embodiment, it is expected that modifications and variations will occur to those skilled in the art upon consideration of the present invention. It is intended that the present invention encompass all such modifications and variations which come with the scope of the claims.

The features disclosed in the foregoing description, in the following claims and/or in the accompanying drawings may, both separately and in any combination thereof, be material for realising the invention in diverse forms thereof.

**Claims**

1. A purified and isolated nucleic acid encoding a TC-CSF and being described by a nucleotide sequence selected from the group consisting of:
a nucleotide sequence encoding a TC-CSF;
a nucleotide sequence which encodes the sequence of amino acids Met-Pro-Asn-Leu-Gly-Ile-Phe-Ala-Asp-Thr-Met-Pro-Glu-Asp-Ala-Gly-Asn-Ser-Val;
a nucleotide sequence which hybridizes with any 20 sequential nucleotides encoding the sequence of amino acids Met-Pro-Asn-Leu-Gly-Ile-Phe-Ala-Asp-Thr-Met-Pro-Glu-Asp-Ala-Gly-Asn-Ser-Val or with the complement thereof;
a nucleotide sequence which hybridizes under strict conditions with any 20 sequential nucleotides in a TC-CSF or with the complement thereof; and
a nucleotide sequence which encodes an epitope encoded by 6 sequential amino acids in a TC-CSF or in the amino acid sequence Met-Pro-Asn-Leu-Gly-Ile-Phe-Ala-Asp-Thr-Met-Pro-Glu-Asp-Ala-Gly-Asn-Ser-Val.

2. The nucleic acid as recited in claim 1 further comprising a vector.

3. The nucleic acid as recited in claim 2 wherein said vector is a cloning vector.

4. The nucleic acid as recited in claim 2 wherein said vector is an expression vector.

5. The nucleic acid as recited in claim 4 further comprising a host cell comprising said vector.

6. The nucleic acid as recited in claim 5 wherein said cell is a eukaryotic cell.

7. The nucleic acid as recited in claim 1 further comprising a host cell comprising said nucleic acid at a location or in a multiplicity in which it is not found in nature.

8. The nucleic acid as recited in claim 7 wherein said cell is a eukaryotic cell.

9. An isolated polypeptide having a biological or immunological property of a TC-CSF and having an amino acid sequence in which at least 6 sequential amino acids are identical to 6 sequential amino acids in a TC-CSF.

10. The isolated polypeptide of claim 9, said polypeptide having an epitope of TC-CSF.

11. The isolated polypeptide of claim 9 wherein said TC-CSF is selected from the group consisting of: human TC-CSF; mouse TC-CSF; rat TC-CSF; bovine TC-CSF; canine TC-CSF; feline TC-CSF; ovine TC-CSF; ape TC-CSF; avian TC-CSF; and porcine TC-CSF.

12. The isolated polypeptide of claim 9 wherein said TC-CSF is an expression product of a cell as recited in claim 5.

13. The isolated polypeptide of claim 9 wherein said TC-CSF is an expression product of a cell as recited in claim 7.

14. The isolated polypeptide as recited in claim 9, further comprising a diluent, adjuvant or carrier.

15. The isolated polypeptide as recited in claim 14 wherein said diluent comprises an isotonic buffer or pharmaceutical grade water for injection to form a pharmaceutical composition.

16. The isolated polypeptide as recited in claim 9, further comprising a label or reporter group.

17. The isolated polypeptide as recited in claim 9. further comprising a support bound to said polypeptide.

18. The isolated polypeptide as recited in claim 9 wherein said polypeptide is associated with a support and an anti-TC-CSF antibody in a package of a diagnostic kit.

19. The isolated polypeptide as recited in claim 9 wherein said polypeptide exhibits a molecular mass of about 55 Kd in SDS-PAGE.

20. An isolated polypeptide as recited in claim 19 having an amino acid sequence comprising Met-Pro-Asn-Leu-Gly-Ile-Phe-Ala-Asp-Thr-Met-Pro-Glu-Asp-Ala-Gly-Asn-Ser-Val.

21. An isolated ligand specifically binding to a TC-CSF.

22. The isolated ligand as recited in claim 21 wherein said ligand is a TC-CSF receptor.

23. The isolated ligand as recited in claim 21 wherein said isolated ligand is an isolated monoclonal or polyclonal antibody exhibiting a specific immunoreactivity with a TC-CSF.

24. The isolated ligand as recited in claim 23 wherein said antibody exhibits a specific immunoreactivity with a polypeptide as recited in claim 9.

25. The isolated ligand as recited in claim 23 wherein said monoclonal or polyclonal antibody exhibits a specific immunoreactivity with a polypeptide as recited in claim 12.

26. The isolated ligand as recited in claim 23 wherein said ligand further comprises serum.

27. An isolated antigen exhibiting immunological characteristics such that it specifically reacts with an isolated ligand as recited in claim 21.

28. An immortalized cell line producing a monoclonal antibody to TC-CSF.

29. A method of treatment comprising the steps of:
administering to cells of a subject in need of hematopoietic therapy a pharmaceutically effective amount of a TC-CSF; and
inducing a TC-CSF hematopoietic reaction.

30. A process for purifying TC-CSF comprising the steps of:
applying cell culture supernatant containing TC-CSF to an anion exchange column; and
collecting a fraction containing a TC-CSF from the HPLC anion exchange column.

31. The process as recited in claim 30 wherein said applying step comprises the step of introducing the TC-CSF onto a column comprising a quaternary ammonium group bound to a support.

32. The process as recited in claim 30, further comprising the steps of:
introducing a fluid comprising TC-CSF into a gel filtration column; and
retaining a fraction comprising TC-CSF.